# EUROPEAN PATENT SPECIFICATION

(11) **EP 3 426 663 B1**
(45) Date of publication and mention of the grant of the patent: **29.01.2020**
(21) Application number: 16815642.0
(22) Date of filing: 15.11.2016
(51) Int. Cl.: C07D 501/04, C07D 501/16

(54) **A PROCESS FOR ALKYLATING THE HYDROXYMETHYL GROUP AT POSITION -3 OF CEPHALOSPORINS**
VERFAHREN ZUR ALKYLIERUNG DER HYDROXYMETHYLGRUPPE AN POSITION -3 VON CEPHALOSPORINEN
PROCÉDÉ D'ALKYLATION DU GROUPE HYDROXYMÉTHYLE EN POSITION -3 DES CÉPHALOSPORINES

(30) Priority: 07.03.2016 IN 201611007853
(43) Date of publication of application: 16.01.2019
(73) Proprietor: Dhanuka Laboratories Ltd., New Delhi 110005 (IN)
(72) Inventor: SINGH, Sandeep, Gurgaon Haryana 122004 (IN); SRIVASTAVA, Alok, Gurgaon Haryana 122004 (IN); DHANUKA, Manish, Gurgaon Haryana 122004 (IN)
(74) Representative: Corizzi, Valérie
(86) International application number: PCT/IB2016/056860
(87) International publication number: WO 2017/153824

(56) References cited:
- US-A- 3 996 216
- US-A- 4 245 088
- US-A- 5 597 914
- US-B1- 6 620 930

## Description

### Field of Invention:

The present invention lies in the field of synthetic chemistry and relates to a process for alkylating the hydroxymethyl group at position -3 of cephalosporin i.e. 7-Amino-3-hydroxymethyl-3-cephem-4-carboxylic acid (D-7-ACA) using an alkylating agent, strong acid and a suitable solvent.

### Background of the Invention:

Cephalosporins are beta-lactam compounds in which the beta-lactam ring is fused to a 6-membered dihydrothiazine ring, thus forming the cephem nucleus. Side chain modifications to the cephem nucleus confers 1) an improved spectrum of antibacterial activity, 2) pharmacokinetic advantages, and 3) additional side effects. Based on their spectrum of activity, cephalosporins can be broadly categorized into four generations. The cephalosporin nucleus can be modified to gain different properties. Cephalosporins are sometimes grouped into "generations" by their antimicrobial properties. The first cephalosporins were designated first-generation cephalosporins, whereas, later, more extended-spectrum cephalosporin's were classified as second-generation cephalosporins. Each newer generation has significantly greater Gram-negative antimicrobial properties than the preceding generation, in most cases with decreased activity against Gram-positive organisms. Fourth-generation cephalosporin's, however, have true broad-spectrum activity. The classification of cephalosporins into "generations" is commonly practiced, although the exact categorization is often imprecise. For example, the fourth generation of cephalosporin's is not recognized as such in Japan.[citation needed] In Japan, cefaclor is classed as a first-generation cephalosporin, though in the United States it is a second-generation one; and cefbuperazone, cefminox, and cefotetan are classed as second-generation cephalosporins. Cefmetazole and cefoxitin are classed as third-generation cephems. Flomoxef and latamoxef are in a new class called oxacephems.

Cephalosporins are indicated for the prophylaxis and treatment of infections caused by bacteria susceptible to this particular form of antibiotic. First-generation cephalosporins are active predominantly against Gram-positive bacteria, and successive generations have increased activity against Gram-negative bacteria (albeit often with reduced activity against Gram-positive organisms).

### Prior Art of the Invention:

JP Patent No. 84,163,387 teaches a method of preparing 7-amino-3-methoxymethyl-3-cephem-4-carboxylic acid by treating 7-aminocephalosporanic acid with methanesulfonic acid-methanol. However, this method also has the problem of low yield (approximately 30%), and the product purity is poor (approximately 30 to 40%) due to the formation of by-products such as lactone or the degradation materials of the β-lactam ring.

EP patent No. 204,657,7-amino-3-methoxymethyl-3-cephem-4-carboxylic acid may be obtained by reacting 7-ACA in sulfolane with boron trifluoride-methanol, but this method requires the use of gaseous boron trifluoride which is hazardous and difficult to handle.

JP Patent No. 88,115,887 and JP Patent No. 89,242,590, describe a process wherein 7-amino-3-methoxymethyl-3-cephem-4-carboxylic acid has been prepared by treating 7-aminocephalosporanic acid with boron trifluoride-methanol in the presence of halosulfonic acid or alkylsulfonic acid or zinc chloride-methanol. These methods give relatively good yields (approximately 60%), but still have the problem of low product purity.

EP patent No. 343,926 teaches a method of preparing 7-amino-3-methoxymethyl-3-cephem-4-carboxylic acid by reacting 7-aminocephalosporanic acid with trimethyl borate in sulfolane, in the presence of sulfuric acid and antimony pentachloride. However, this method requires the use of expensive antimony pent chloride as well as 98% trimethyl borate which is difficult to handle.

EP patent No. 485,204 teaches a method for preparing 7-amino-3-methoxymethyl-3-cephem-4-carboxylic acid by treating 7-ACA in a solution containing an alkoxysulfonic acid with a trialkyl borate and CH₂(OR)₂. However, this method still has to deal with the difficulty of handling 98% trimethyl borate and also suffers from the problem of poor process controllability.

US Patent No. 6620930 B1: 7-amino-3-methoxymethyl-3-cephem-4-carboxylic acid has been prepared by treating 7-Aminocephalosporanic acid with azeotropic mixture of Trimethylborate in methanol in the presence of methane sulphonic acid. These methods give relatively good yields (approximately 60%), but still have the problem of low product quantitative purity of cefpodoxime acid.

In EP 0 262 744, 7-ACA or a protected form thereof is reacted with an alcohol in the presence of an excess of Lewis acids, such as SbCl5, BiCl3, FeCl3 or ZnCl2 to form 7-amino-3-alkoxymethyl-3-cephem-4-carboxylic acid. However, only low yields are obtained. The replacement of the alcohol with trialkyl borate or trialkyl orthoformate and the use of the above-mentioned catalysts only leads to a slight increase in yield as relevant in EP0343926.

AT 384 222 (EP 0 204 657), describe the reaction of 7-ACA with BF3/methanol in sulpholane, in a large excess of BF3 and high reaction temperatures are required. Only low yields and poor quality of the 7-amino-3-meth oxymethyl-3-cephem-4-carboxylic acid (7-AMCA) are obtained. and Japanese patent application 63/115887 describes a variant in which the highly poisonous Fluorosulphonic acid is additionally, employed, without effecting a decisive improvement in the process.

JP 59/163387 and EP 0 442 385 When using other catalysts, such as sulphuric acid, methanesulphonic acid or trifluoromethane sulphonic acid, the compounds of 7-amino-3-methoxymethyl-3-cephem-4-carboxylic acid are obtained in yields of only around 50%.

Japanese patent application 57/192392 and in DE 3,244,457 utilizes reaction of protected 7-ACA derivatives is described in. Here, the appropriately protected compounds of formula I are obtained only in moderate yields. Also, additional reaction steps are required to protect and deprotect the starting and end products. AT 303 955 (GB 1,241,657) describes lower yields obtained if a protected 3-halomethyl compound is used as the starting compound to produce the appropriately protected 3-alkoxymethyl compound.

AT 306 240 (GB 1,241,656) reveals the process commencing with a 7-amino-3-hydroxymethyl-3-cephem-4-carboxylic acid which is protected at the carboxylic acid group and at the amino group, and is then methylated in the presence of BF3/diethyletherate at the hydroxymethyl function with diazomethane, which is hazardous for reasons of industrial safety.

IN US patent 5,597,914, 7-amino-3-methoxymethyl-3-cephem-4-carboxylic acid has been prepared by treating 7-Amino-3-hydroxy-3-cephem carboxylic acid with dimethoxycarbenium-tetrafluborate in formic acid methyl ester, formic acid ethyl ester, dimethyl carbonate, dipropyl carbonate, nitromethane, sulpholane, dichloromethane, dimethyl sulphoxide or a mixture thereof. These methods give relatively good yields (approximately 77.8%), but this method requires the use of gaseous boron trifluoride which is hazardous and difficult to handle.

Most of the processes reported in the prior art for the preparation of 7-Amino-3-methoxymethyl-3-cephem-4-carboxylic acid (7-AMCA) was from 7-Amino cephalosporanic acid as a starting material in presence of a solvent, an organic acid and an alkylating agent. These processes have led to undesirable products along with the desired product 7-Amino-3-methoxymethyl-3-cephem-4-carboxylic acid (7-AMCA) in moderate yield.

In EP0908461 A1, 7-Amino-3-hydroxymethyl-3-cephem-4-carboxylic acid (D-7-ACA) has been used as a starting material to react with dioxycarbenium-tetrafluoroborate as an alkylating agent to yield 7-Amino-3-methoxymethyl-3-cephem-4-carboxylic acid (7-AMCA).

However, it has never been reported in the prior art to provide an efficient process for the preparation of 7-Amino-3-methoxymethyl-3-cephem-4-carboxylic acid (7-AMCA) starting from 7-Amino-3-hydroxymethyl-3-cephem-4-carboxylic acid (D-7-ACA) using alkylating agent in presence of strong organic acid and a suitable solvent so that 7-AMCA is obtained with increased yield and reduced lactone content.

The present invention provides an efficient process for the preparation of 7-Amino-3-methoxymethyl-3-cephem-4-carboxylic acid (7-AMCA), wherein 7-Amino-3-hydroxymethyl-3-cephem-4-carboxylic acid (D-7-ACA) is used as a starting material to react with suitable alkylating agent in presence of a strong acid and a suitable solvent to provide a yield of 81.0 % having lactone content of 0.2%.

### Objects of the Invention:

Primary object of the invention is to provide a process for the preparation of 7-Amino-3- methoxymethyl-3-cephem-4-carboxylic acid (7-AMCA) by alkylation of 7-Amino-3-hydroxymethyl-3- cephem-4-carboxylic acid (D-7-ACA) in presence of a strong organic acid.

Another object of the invention is to reduce the formation of undesired lactone compound during alkylation.

Yet another object of the present invention is production of cephalosporins with increased yield.

### Summary of the Invention:

A process for alkylating the hydroxymethyl group at position - 3 of 7 Amino-3-hydroxymethyl-3-cephem-4-carboxylic acid (D-7-ACA) using an alkylating agent, strong acid and a suitable solvent. More particularly the invention relates to the preparation of 7-Amino-3-methoxymethyl-3-cephem-4-carboxylic acid (7-AMCA) by alkylation starting from 7-Amino-3-hydroxymethyl-3-cephem-4-carboxylic acid (D-7-ACA). The present process leads to enhanced yield of upto 81% and having reduced lactone content of about 0.2%.

### Detailed Description of the invention:

The present invention relates to a process for the preparation of 7-Amino 3-methoxymethyl-3-cephem-4-carboxylic acid (7-AMCA) having enhanced yield and reduced lactone content, starting from 7-Amino-3-hydroxymethyl-3-cephem-4-carboxylic acid (D-7-ACA) in presence of suitable alkylating agent in presence of a strong acid and a suitable solvent.

More precisely, the present invention relates to a process for alkylating hydroxymethyl group at position - 3 of cephalosporins, the said process comprising the steps of:
a) reacting 3- Hydroxymethyl cephalosporin's;
b) with an alkylating agent selected from a group comprising Formic acid ethyl ester, Formic acid methyl ester, Trialkylorthoformate, and mixtures thereof;
c) in an organic solvent(s) selected from a group consisting of Dialkyl carbonate, Dialkyl dicarbonate, Acetonitrile, Dichloromethane, Sulfolane and mixture thereof;
in presence of an organic acid selected from alkyl sulphonic acid, aryl sulphonic acid and mixtures thereof.

The application also discloses a process wherein alkylating agent used is selected from a group consisting of Formic acid ethyl ester, Formic acid ester methyl ester, Dimethyl sulfate, Trialkyl borate, Trialkyl borate and methanol azeotrope, Trialkylorthoformate, Dioxycarbenium tetrafluborate and mixtures thereof.

The application also discloses a process wherein strong acid used is selected from a group consisting of Boron trifluoride, Boron trifluoride etherate, mineral acid, halosulphonic acid, alkoxy sulphonic acid, Alkyl sulphonic acid and Aryl sulphonic acid and mixtures thereof.

The application also discloses a process wherein the solvent used can be selected from a group consisting of Dimethyl formamide, Dimethyl acetamide, Dimethyl sulphoxide, Dialkyl carbonate, Dialkyl dicarbonate, Chlorinated solvent, aromatic hydrocarbon, Alkyl formate, Sulpholane, etheral solvent and mixture thereof.

In still another embodiment of the present invention, the work up of alkylated reaction product can be carried out in either aqueous or non- aqueous medium. The process for alkylating hydroxymethyl group at position-3 of cephalosporins, comprised reacting 3-Hydroxymethyl cephalosporin's with a suitable alkylating agent in a suitable organic solvent(s) in presence of an organic acid. The alkylation introduces an alkyl group for the production of cephalosporin compound of formula-I.

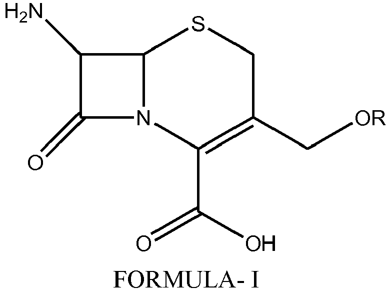

Wherein R is defined as alkyl or substituted alkyl group
Comprising the step of reacting a compound of formula - II 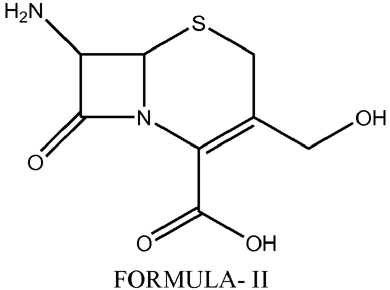 in a solvent with an alkylating agent in presence of an organic acid.

R in formula-I is defined as substituted alkyl group or an unsubstituted alkyl group, wherein R is defined as methyl or ethyl group. The process of the subject invention is effected in an organic solvent selected from a group comprising of Dialkyl carbonate, Dialkyl dicarbonate, Acetonitrile, Dichloromethane, Sulpholane, and mixtures thereof. The application discloses a process wherein alkylating agent can be selected from a group comprising of Formic acid ethyl ester, Formic acid ester methyl ester, Dimethyl sulfate, Trialkyl borate, trialkyl borate and methanol azeotrope, Trialkylorthoformate, Dioxycarbenium-tetrafluborate and mixtures thereof.

The application also discloses a process wherein the alkylating agent can be a methylating selected from a group comprising of Trimethyl orthoformate, Trimethyl borate, Trimethyl borate: Methanol azeotropic mixture, methyl formate, Dimethoxy methane, Dimethyl sulfate, Dimethoxycarbenium- tetrafluborate, Diethoxycarbenium- tetrafluborate. The application also discloses that the process can be effected in the presence of an organic acid selected from a group consisting of alkyl sulphonic acid, BF3 Etherate, Boron trifluoride, Methoxy sulphonic acid, Aryl sulphonic acid. The process of the present invention is effected in a solvent such as Dichloromethane, Sulpholane, Dimethyl carbonate, dimethyldicarbonate, diethylcarbonate, diethyldicarbonate and mixtures thereof. The process of the present invention is effected in temperature range of (-)40°C to (+)40°C.

In another embodiment, the process of the present invention is effected in presence of trimethylorthoformate.

In yet another embodiment, the process of the present invention is effected in the presence of methane sulphonic acid and Benzene sulphonic acid.

### EXAMPLES

**The invention is illustrated with the following examples 1-6 and 12-19 which should however not be construed to limit the scope of the present invention.**

### Example 1

**(A)** 7-Amino-3-Hydroxymethyl-3-cephem-4-carboxylic acid (100 gm,0.4347 mol) is added to mixture of Dimethyl carbonate (300 ml) and Trimethyl orthoformate (96.8 gm,2.1mol) at a temperature ranging between 5°-10°C followed by addition of Methane sulphonic acid (200.0 gm, 4.79 mol) The reaction mixture stirred for 20-35 min around 30-40°C, checked the reaction on HPLC. The lactone percentage is around 6-15% with respect to 7-Amino-3-methoxymethyl-3-cephem-4-carboxylic acid (7-AMCA) and quenched the reaction mass by adding it to chilled brine (300ml) and separated the layers. Added aqueous ammonia solution (206 gm 20% ammonia) slowly over a period of 60-120min maintaining the temperature in the range of 10-30°C to lower Aqueous layer till the pH of the mass attained 3.3 to 3.5. The precipitated 7-Amino-3-methoxymethyl-3-cephem-4-carboxylic acid (7-AMCA) is isolated wet yield-150gm, LOD- 46%.
**(B)** 7-Amino-3-methoxymethyl-3-cephem-4-carboxylic acid (150 gm) obtained in step A above is taken in an aqueous methanol and cooled to 15-20°C and treated with Benzothiazol-2-yl (Z)-2-methoxyimino-2-(2-aminothiazole-4-yl) thioacetate (MAEM) in the presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (118 gm, Assay 98.8%).

### Example 2

**(A)** 7-Amino-3-Hydroxymethyl-3-cephem-4-carboxylic acid (100 gm,0.4347 mol) is added to mixture of Dimethyl carbonate (300 ml) and Trimethyl orthoformate (96.8 gm, 2.1 mol) at a temperature ranging between 5°-10°C followed by addition of Benzene sulphonic acid (329.0 gm, 4.79 mol) The reaction mixture stirred for 20-35 min around 30-40°C, checked the reaction on HPLC. The lactone percentage is around 6-15% with respect to 7-Amino-3-methoxymethyl-3-cephem-4-carboxylic acid (7-AMCA) and quenched the reaction mass by adding it to chilled brine (300 ml) and separated the layers. Added aqueous ammonia solution (206 gm 20% ammonia) slowly over a period of 60-120min maintaining the temperature in the range of 10-30°C to lower Aqueous layer till the pH of the mass attained 3.3 to 3.5. The precipitated 7-Amino-3-methoxymethyl-3-cephem-4-carboxylic acid (7-AMCA) is isolated. Wet yield-146 gm, LOD- 46%.
**(B)** 7-Amino-3-methoxymethyl-3-cephem-4-carboxylic acid (146 gm) obtained in step A above is taken in an aqueous methanol and cooled to 15-20°C and treated with Benzothiazol-2-yl (Z)-2-methoxyimino-2-(2-aminothiazole-4-yl) thioacetate (MAEM) in the presence of Tri ethylamine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (113 gm, Assay 98.6%).

### Example 3

**(A)** 7-Amino-3-Hydroxymethyl-3-cephem-4-carboxylic acid (100 gm, 0.4347 mol) is added to mixture of Sulfolane (300 ml) and Trimethyl orthoformate (96.8 gm, 2.1 mol) at a temperature ranging between 5°-10°C, then add Methane sulphonic acid (200.0 gm, 4.79 mol) The reaction mixture stirred for 20-35 min around 30-40°C, check the reaction on HPLC. The lactone percentage is around 6-15% with respect to 7-Amino-3-methoxymethyl-3-cephem-4-carboxylic acid (7-AMCA) and quenched the reaction mass by adding it to chilled brine (300ml). Added aqueous ammonia solution (206 gm 20% ammonia) slowly over a period of 60-120 min maintaining the temperature in the range of 10-30°C till the pH of the mass up to 3.3 to 3.5. The precipitated 7-Amino-3-methoxymethyl-3-cephem-4-carboxylic acid (7-AMCA) is isolated. Wet yield-140gm, LOD - 44%.
**(B)** 7-Amino-3-methoxymethyl-3-cephem-4-carboxylic acid (140 gm) obtained in step A above is taken in an aqueous methanol and cooled to 15-20°C and treated with Benzothiazol-2-yl (Z)-2-methoxyimino-2-(2-aminothiazole-4-yl) thioacetate (MAEM) in presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (112 gm, Assay 98.5%).

### Example 4

**(A)** 7-Amino-3-Hydroxymethyl-3-cephem-4-carboxylic acid (100 gm,0.4347 mol) is added to mixture of Dichloromethane (300ml) and Trimethyl orthoformate (96.8 gm, 2.1 mol) at a temperature ranging between 5°-10°C, then add Methane sulphonic acid (200.0 gm, 4.79 mol). The reaction mixture stirred for 20-35 min around 30-40°C and checked the reaction on HPLC. The lactone percentage was recorded around 6-15% with respect to 7-Amino-3-methoxymethyl-3-cephem-4-carboxylic acid (7-AMCA) and quenched the reaction mass by adding it to chilled brine (300 ml), Separated the aqueous layer, added aqueous ammonia solution (206 gm 20% ammonia) slowly over a period of 60-120min maintaining the temperature in the range of 10-30°C in lower aqueous layer till the pH of the mass reached to 3.3 to 3.5. The precipitated 7-Amino-3-methoxymethyl-3-cephem-4-carboxylic acid (7-AMCA) is isolated having a wet yield of 132 gm, LOD - 40.0%.
**(B)** 7-Amino-3-methoxymethyl-3-cephem-4-carboxylic acid (132 gm) obtained in step A above is taken in an aqueous methanol and cooled to 15-20°C and treated with Benzothiazol-2-yl (Z)-2-methoxyimino-2-(2-aminothiazole-4-yl) thioacetate (MAEM) in presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (111.0 gm, Assay 98.4%).

### Example 5

**(A)** 7-Amino-3-Hydroxymethyl-3-cephem-4-carboxylic acid (100 gm,0.4347 mol) is added to a mixture of Acetonitrile (300 ml) and Trimethyl orthoformate (96.8 gm, 2.1 mol) at a temperature ranging between 5°-10°C followed by addition of Methane sulphonic acid (200.0 gm, 4.79 mol). The reaction mixture is stirred for 20-35 min around 30-40°C and checked on HPLC. The lactone percentage was recorded around 6-15% with respect to 7-Amino-3-methoxymethyl-3-cephem-4-carboxylic acid (7-AMCA) and quenched the reaction mass by adding it to chilled brine (300 ml). Added aqueous ammonia solution (206 gm 20% ammonia) slowly over a period of 60-120 min maintaining the temperature in the range of 10-30°C till the pH of the mass up to 3.3 to 3.5. The precipitated 7-Amino-3-methoxymethyl-3-cephem-4-carboxylic acid (7-AMCA) is isolated. Wet yield-138gm, LOD - 43%.
**(B)** 7-Amino-3-methoxymethyl-3-cephem-4-carboxylic acid (138 gm) obtained in step A above is taken in an aqueous methanol and cooled to 15-20°C and treated with Benzothiazol-2-yl (Z)-2-methoxyimino-2-(2-aminothiazole-4-yl) thioacetate (MAEM) in presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (111gm, Assay 98.3%).

### Example 6

**(A)** 7-Amino-3-Hydroxymethyl-3-cephem-4-carboxylic acid (100 gm,0.4347 mol) is added to a mixture of Sulfolane (150 ml), Dimethyl carbonate (150 ml) and Trimethyl orthoformate (96.8 gm, 2.1 mol) at a temperature ranging between 5°-10°C followed by addition of Methane sulphonic acid (200.0 gm, 4.79 mol). The reaction mixture stirred for 20-35 min around 30-40°C and checked the reaction on HPLC. The lactone percentage is around 6-15% with respect to 77-Amino-3-methoxymethyl-3-cephem-4-carboxylic acid (7-AMCA) and quenched the reaction mass by adding it to chilled brine (300 ml) and separated the layer. Added aqueous ammonia solution (206 gm 20% ammonia) slowly over a period of 60-120 min maintaining the temperature in the range of 10-30°C till the pH of the mass reached up to 3.3 to 3.5. The precipitated 7-Amino-3-methoxymethyl-3-cephem-4-carboxylic acid (7-AMCA) is isolated. Wet yield-138gm, LOD-42.5%.
**(B)** 7-Amino-3-methoxymethyl-3-cephem-4-carboxylic acid (138 gm) obtained in step A above is taken in an aqueous methanol and cooled to 15-20°C and treated with Benzothiazol-2-yl (Z)-2-methoxyimino-2-(2-aminothiazole-4-yl) thioacetate (MAEM) in the presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (112 gm, Assay 98.4%).

### Example 7 (reference example)

**(A)** 7-Amino-3-Hydroxymethyl-3-cephem-4-carboxylic acid (100 gm,0.4347 mol) is added to a mixture of Dimethyl carbonate (300 ml) and Trimethyl borate (94.8 gm, 2.1 mol) at a temperature ranging between 5°-10°C followed by addition of Methane sulphonic acid (200.0 gm, 4.79 mol). The reaction mixture stirred for 20-35 min around 30-40°C and checked the reaction on HPLC. The lactone percentage is around 30-45% with respect to 7-Amino-3-methoxymethyl-3-cephem-4-carboxylic acid (7-AMCA) Reaction is not taken further due to the formation high percentage of undesirable lactone product.

### Example 8 (reference example)

**(A)** 7-Amino-3-Hydroxymethyl-3-cephem-4-carboxylic acid (100 gm,0.4347 mol) is added to a mixture of Dimethyl carbonate (300 ml) and Trimethyl borate: Methanol Azeotropic mixture (135.5 gm, 2.1 mol) at a temperature ranging between 5°-10°C followed by addition of Methane sulphonic acid (200.0gm, 4.79 mol). The reaction mixture stirred for 20-35 min around 30-40°C and checked the reaction on HPLC. The lactone percentage is around 60-70% with respect to 7-Amino-3-methoxymethyl-3-cephem-4-carboxylic acid (7-AMCA). Reaction is not taken further due to the formation high percentage of undesirable lactone product.

### Example 9 (reference example)

**(A)** 7-Amino-3-Hydroxymethyl-3-cephem-4-carboxylic acid (100 gm,0.4347 mol) is added to a mixture of Dimethyl carbonate (300 ml) and Dimethyl sulfate (115.0 gm, 2.1 mol) at a temperature ranging between 5°-10°C followed by addition of Methane sulphonic acid (200.0 gm, 4.79mol). The reaction mixture stirred for 20-35 min around 30-40°C and checked the reaction on HPLC. The lactone percentage is around 80-90% with respect to 7-Amino-3-methoxymethyl-3-cephem-4-carboxylic acid (7-AMCA). Reaction is not taken further due to the formation high percentage of undesirable lactone product.

### Example 10 (reference example)

**A)** 7-Amino-3-Hydroxymethyl-3-cephem-4-carboxylic acid (100 gm,0.4347 mol) is added to a mixture of Dimethyl carbonate (300ml) and Methyl formate (54.8 gm, 2.1 mol) at a temperature ranging between 5°-10°C followed by addition of Methane sulphonic acid (200.0 gm, 4.79 mol). The reaction mixture stirred for 20-35 min around 30-40°C and checked the reaction on HPLC. The lactone percentage is around 45-50% with respect to 7-Amino-3-methoxymethyl-3-cephem-4-carboxylic acid (7-AMCA). Reaction is not taken further due to the formation high percentage of undesirable lactone product.

### Example 11 (reference example)

**(A)** 7-Amino-3-Hydroxymethyl-3-cephem-4-carboxylic acid (100 gm,0.4347 mol) is added to a mixture of Dimethyl carbonate (300ml) and Dimethoxymethane (69.5 gm, 2.1 mol) at a temperature ranging between 5°-10°C followed by addition of Methane sulphonic acid (200.0 gm, 4.79 mol). The reaction mixture stirred for 20-35 min around 30-40°C and checked the reaction on HPLC. The lactone percentage is around 40-55% with respect to 7-Amino-3-methoxymethyl-3-cephem-4-carboxylic acid (7-AMCA). Reaction is not taken further due to the formation high percentage of undesirable lactone product.

### Example 12

**(A)** 7-Amino-3-Hydroxymethyl-3-cephem-4-carboxylic acid (100 gm,0.4347 mol) is added to a mixture of Dimethyl dicarbonate (300ml) and Trimethyl orthoformate (96.8 gm, 2.1 mol) at a temperature ranging between 5°-10°C followed by addition of Methane sulphonic acid (200.0 gm, 4.79 mol). The reaction mixture stirred for 20-35 min around 30-40°C and checked the reaction on HPLC. The lactone percentage is around 6-15% with respect to 7-Amino-3-methoxymethyl-3-cephem-4-carboxylic acid (7-AMCA) and quenched the reaction mass by adding it to chilled brine (300 ml) and separate the layer. Added aqueous ammonia solution (206 gm 20% ammonia) slowly over a period of 60-120min maintaining the temperature in the range of 10-30°C in lower Aqueous layer till the pH of the mass up to 3.3 to 3.5. The precipitated 7-Amino-3-methoxymethyl-3-cephem-4-carboxylic acid (7-AMCA) is isolated. wet yield-145gm, LOD- 44%.
**(B)** 7-Amino-3-methoxymethyl-3-cephem-4-carboxylic acid (145 gm) obtained in step A above is taken in aqueous methanol and cooled to 15-20°C and treated with Benzothiazol-2-yl (Z)-2-methoxyimino-2-(2-aminothiazole-4-yl) thioacetate (MAEM) in presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (114 gm, Assay 98.7%).

### Example 13

**(A)** 7-Amino-3-Hydroxymethyl-3-cephem-4-carboxylic acid (100 gm,0.4347 mol) is added to a mixture of Dimethyl carbonate (300 ml) and Trimethyl orthoformate (96.8 gm, 2.1 mol) at a temperature ranging between 5°-10°C followed by addition of BF3 (141.6 gm, 4.79 mol). The reaction mixture stirred for 60-90 min around 30-40° and checked the reaction on HPLC. The lactone percentage is around 35-40% with respect to 7-Amino-3-methoxymethyl-3-cephem-4-carboxylic acid (7-AMCA). Reaction is not taken further due to the formation high percentage of undesirable lactone product.

### Example 14

**(A)** 7-Amino-3-Hydroxymethyl-3-cephem-4-carboxylic acid (100 gm,0.4347 mol) is added to a mixture of Dimethyl carbonate (300ml) and Trimethyl orthoformate (96.8 gm, 2.1 mol) at a temperature ranging between 5°-10°C followed by addition of Methoxy sulphonic acid (233.2 gm, 4.79 mol). The reaction mixture stirred for 60-90 min around 30-40°C and checked the reaction on HPLC. The lactone percentage is around 35-40% with respect to 7-Amino-3-methoxymethyl-3-cephem-4-carboxylic acid (7-AMCA). Reaction is not taken further due to the formation high percentage of undesirable lactone product.

### Example 15

**(A)** 7-Amino-3-Hydroxymethyl-3-cephem-4-carboxylic acid (100 gm, 0.4347 mol) is added to a mixture of Dimethyl carbonate (300 ml) and Trimethyl orthoformate (96.8 gm, 2.1 mol) at a temperature ranging between 5°-10°C followed buy addition of Methane sulphonic acid (100.0gm, 2.39 mol). The reaction mixture stirred for 90-150 min around 30-40°C and checked the reaction on HPLC. The lactone percentage is around 40-45% with respect to 7-Amino-3-methoxymethyl-3-cephem-4-carboxylic acid (7-AMCA). Reaction is not taken further due to the formation high percentage of undesirable lactone product.

### Example 16

**(A)** 7-Amino-3-Hydroxymethyl-3-cephem-4-carboxylic acid (100 gm, 0.4347 mol) is added to mixture of Dimethyl carbonate (300ml) and Trimethyl orthoformate (96.8 gm, 2.1 mol) at a temperature ranging between 5°-10°C, then add Methane sulphonic acid (300.0 gm, 7.18 mol) The reaction mixture stirred for 20-35 min around 30-40°C, check the reaction on HPLC. The lactone percentage is around 50-55% with respect to 7-Amino-3-methoxymethyl-3-cephem-4-carboxylic acid (7-AMCA). Reaction is not taken further due to the formation high percentage of undesirable lactone product.

### Example 17

**(A)** 7-Amino-3-Hydroxymethyl-3-cephem-4-carboxylic acid (100 gm, 0.4347 mol) is added to mixture of Dimethyl carbonate (300 ml) and Trimethyl orthoformate (69. 2 gm, 1.50 mol) at a temperature ranging between 5°-10°C followed by addition of Methane sulphonic acid (200.0gm, 4.79 mol). The reaction mixture stirred for 30-45 min around 30-40°C, check the reaction on HPLC. The lactone percentage is around 25-30% with respect to 7-Amino-3-methoxymethyl-3-cephem-4-carboxylic acid (7-AMCA). Reaction is not taken further due to the formation high percentage of undesirable lactone product.

### Example 18

**(A)** 7-Amino-3-Hydroxymethyl-3-cephem-4-carboxylic acid (100 gm, 0.4347 mol) is added to mixture of Dimethyl carbonate (300 ml) and Trimethyl orthoformate (138.2 gm, 3.0 mol) at a temperature ranging between 5°-10°C followed by addition of Methane sulphonic acid (200.0 gm, 4.79 mol). The reaction mixture stirred for 20-35 min around 30-40°C and checked the reaction on HPLC. The lactone percentage is around 12-18% with respect to 7-Amino-3-methoxymethyl-3-cephem-4-carboxylic acid (7-AMCA). and quenched the reaction mass by adding it to chilled brine (300ml) and separate the layer. Added aqueous ammonia solution (206 gm 20% ammonia) slowly over a period of 60-120 mins maintaining the temperature in the range of 10-30°C in lower Aqueous layer till the pH of the mass up to 3.3 to 3.5. The precipitated 7-Amino-3-methoxymethyl-3-cephem-4-carboxylic acid (7-AMCA) is isolated. wet yield-135m, LOD- 42.0%.
**(B)** 7-Amino-3-methoxymethyl-3-cephem-4-carboxylic acid (135 gm) obtained in step A above is taken in an aqueous methanol and cooled to 15-20°C and treated with Benzothiazol-2-yl (Z)-2-methoxyimino-2-(2-aminothiazole-4-yl) thioacetate (MAEM) in presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (109 gm, Assay 98.2%).

### Example 19

**(A)** 7-Amino-3-Hydroxymethyl-3-cephem-4-carboxylic acid (100 gm,0.4347 mol) is added to mixture of Dimethyl carbonate (300 ml) and Trimethyl orthoformate (96.8 gm, 2.1 mol) at a temperature ranging between 5°-10°C followed by addition of Methane sulphonic acid (200.0 gm, 4.79 mol). The reaction mixture stirred for 20-35 min around 30-40°C and checked the reaction on HP LC. The lactone percentage is around 6-15% with respect to 7-Amino-3-methoxymethyl-3-cephem-4-carboxylic acid (7-AMCA) and quenched the reaction mass by adding it to chilled Methanol (300ml) then adjust pH 0.5-0.6 by adding methanolic ammonia, salt is precipitated. Filtered the salt and added Trimethyl amine (40 ml) slowly over a period of 60-120 mins maintaining the temperature in the range of 10-20°C in filtrate mass till the pH of the mass up to 3.3 to 3.5. The precipitated 7-Amino-3-methoxymethyl-3-cephem-4-carboxylic acid (7-AMCA) is isolated. wet yield-110gm, LOD- 25%.
**(B)** 7-Amino-3-methoxymethyl-3-cephem-4-carboxylic acid (110gm) obtained in step A above is taken in a aqueous methanol and cooled to 15-20°C and treated with Benzothiazol-2-yl (Z)-2-methoxyimino-2-(2-aminothiazole-4-yl) thioacetate (MAEM) in presence of Tri ethyl amine and stirred further for 5-6 hours. The reaction mixture is quenched by adding water, adjusting the pH to around 5.5 to 6.0 by using dilute sulphuric acid. Filtering the solid obtained and treating the filtrate with activated charcoal, followed by filtration and adjusting the pH of the filtrate to 2.6 to 2.8 by using dilute sulphuric acid to obtain cefpodoxime acid (120 gm, Assay 98.7%).

## Claims

1. A process for alkylating hydroxymethyl group at position - 3 of cephalosporins, the said process comprising the steps of:
a) reacting 3- Hydroxymethyl cephalosporin's;
b) with an alkylating agent selected from a group comprising Formic acid ethyl ester, Formic acid methyl ester, Trialkylorthoformate, and mixtures thereof;
c) in an organic solvent(s) selected from a group consisting of Dialkyl carbonate, Dialkyl dicarbonate, Acetonitrile, Dichloromethane, Sulfolane and mixture thereof;
in presence of an organic acid selected from alkyl sulphonic acid, aryl sulphonic acid and mixtures thereof.

2. The process as claimed in claim 1 to produce cephalosporin compound of formula 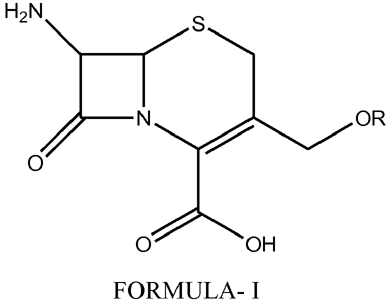 Wherein R is defined as alkyl or substituted alkyl group
Comprising the step of reacting a compound of formula - II 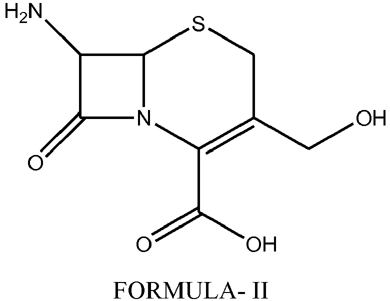 in a solvent with an alkylating agent in presence of an organic acid.

3. The process as claimed in claim 1 to 2, wherein R is defined as substituted alkyl group.

4. The process as claimed in claim 1 to 2, wherein R is defined as unsubstituted alkyl group.

5. The process as claimed in claim 4, wherein R is defined as methyl or ethyl group.

6. The process as claimed in claim 1 to 5, wherein the alkylating agent is a methylating agent.

7. The process as claimed in claim 1, wherein the process is effected in the presence of trimethylorthoformate.

8. The process as claimed in claim 1, wherein the process is effected in the presence of methane sulphonic acid and Benzene sulphonic acid.

9. The process as claimed in claims 1 to 8, wherein the process is effected in temperature range of (-)40°C to (+)40°C.

## Patentansprüche

1. Verfahren zur Alkylierung einer Hydroxymethylgruppe in der 3-Position von Cephalosporinen, wobei das Verfahren die folgenden Schritte umfasst:
a) Umsetzen von 3-Hydroxymethylcephalosporinen
b) mit einem Alkylierungsmittel aus der Gruppe bestehend aus Ameisensäureethylester, Ameisensäuremethylester, Orthoameisensäuretrialkylester und Mischungen davon
c) in einem oder mehreren organischen Lösungsmitteln aus der Gruppe bestehend aus Dialkylcarbonat, Dialkyldicarbonat, Acetonitril, Dichlormethan, Sulfolan und einer Mischung davon
in Gegenwart einer aus Alkylsulfonsäure, Arylsulfonsäure und Mischungen davon ausgewählten organischen Säure.

2. Verfahren nach Anspruch 1 zur Herstellung einer Cephalosporinverbindung der Formel 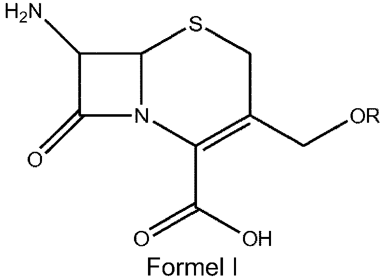 wobei R als Alkyl oder substituierte Alkylgruppe definiert ist, umfassend den Schritt des Umsetzens einer Verbindung der Formel II 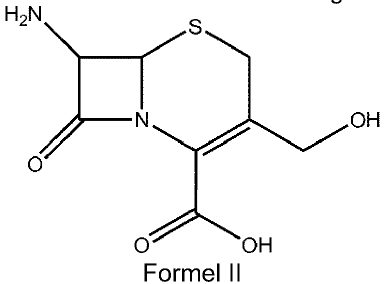 in einem Lösungsmittel mit einem Alkylierungsmittel in Gegenwart einer organischen Säure.

3. Verfahren nach Anspruch 1 bis 2, wobei R als substituierte Alkylgruppe definiert ist.

4. Verfahren nach Anspruch 1 bis 2, wobei R als unsubstituierte Alkylgruppe definiert ist.

5. Verfahren nach Anspruch 4, wobei R als Methyl- oder Ethylgruppe definiert ist.

6. Verfahren nach Anspruch 1 bis 5, wobei es sich bei dem Alkylierungsmittel um ein Methylierungsmittel handelt.

7. Verfahren nach Anspruch 1, wobei das Verfahren in Gegenwart von Orthoameisensäuretrimethylester durchgeführt wird.

8. Verfahren nach Anspruch 1, wobei das Verfahren in Gegenwart von Methansulfonsäure und Benzolsulfonsäure durchgeführt wird.

9. Verfahren nach den Ansprüchen 1 bis 8, wobei das Verfahren im Temperaturbereich von (-)40 °C bis (+)40 °C durchgeführt wird.

## Revendications

1. Procédé pour l'alkylation du groupe hydroxyméthyle au niveau d'une position - 3 de céphalosporines, ledit procédé comprenant les étapes de :
a) mise en réaction de 3-hydroxyméthyl céphalosporines ;
b) avec un agent d'alkylation choisi dans un groupe comprenant l'ester éthylique de l'acide formique, l'ester méthylique de l'acide formique, un orthoformiate de trialkyle, et des mélanges correspondants ;
c) dans un ou plusieurs solvants organiques choisis dans le groupe constitué par un carbonate de dialkyle, un dicarbonate de dialkyle, l'acétonitrile, le dichlorométhane, le sulfolane et un mélange correspondant ;
en présence d'un acide organique choisi parmi un acide alkylsulfonique, un acide arylsulfonique et des mélanges correspondants.

2. Procédé selon la revendication 1 pour produire un composé de céphalosporine de formule 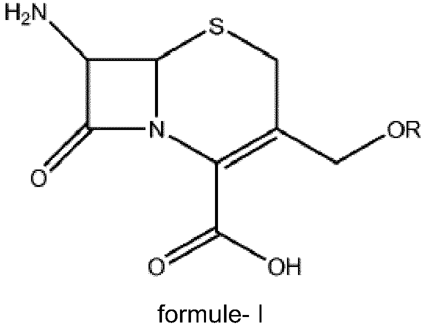 R étant défini comme un groupe alkyle ou alkyle substitué comprenant l'étape de mise en réaction d'un composé de formule - Il 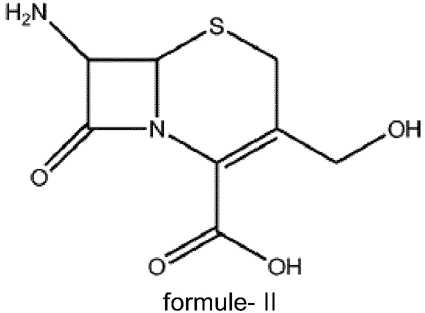 dans un solvant avec un agent d'alkylation en présence d'un acide organique.

3. Procédé selon la revendication 1 et 2, R étant défini comme un groupe alkyle substitué.

4. Procédé selon la revendication 1 et 2, R étant défini comme un groupe alkyle non substitué.

5. Procédé selon la revendication 4, R étant défini comme un groupe méthyle ou éthyle.

6. Procédé selon la revendication 1 à 5, l'agent d'alkylation étant un agent de méthylation.

7. Procédé selon la revendication 1, le procédé étant réalisé en présence d'orthoformiate de triméthyle.

8. Procédé selon la revendication 1, le procédé étant réalisé en présence d'acide méthanesulfonique et d'acide benzènesulfonique.

9. Procédé selon les revendications 1 à 8, le procédé étant réalisé dans une plage de température de (-)40 °C à (+)40 °C.
